# EUROPEAN PATENT APPLICATION

(11) **EP 4 421 172 A1**
(43) Date of publication of application: **28.08.2024**
(21) Application number: 22897771.6
(22) Date of filing: 21.11.2022
(51) Int. Cl.: C12N 15/11, C12Q 1/6869

(54) **ADAPTER, COMPLEX, SINGLE-STRANDED MOLECULE, KIT, METHOD, AND APPLICATION**

(30) Priority: 23.11.2021 CN 202111398053
(71) Applicant: Qitan Technology Ltd., Chengdu, Chengdu, Sichuan 610041 (CN)
(72) Inventor: LIU, Xianyu, Chengdu, Sichuan 610041 (CN); CHANG, Xin, Chengdu, Sichuan 610041 (CN)
(74) Representative: Boult Wade Tennant LLP
(86) International application number: PCT/CN2022/133303
(87) International publication number: WO 2023/093688

(57) **Abstract**

Provided are an adapter for characterizing biomolecules, a complex, a single-stranded molecule, a kit, a method, and use thereof. The adapter comprises a main body strand and a connecting strand, wherein the main body strand is used for binding to a biomolecule to be tested and a polynucleotide-binding protein; the connecting strand comprises a connecting region and a side chain region, the connecting region being connected to the main body strand, and the side chain region being used for connecting to an external connector; the binding ability of the side chain region to the polynucleotide-binding protein is weaker than the binding ability of the main body strand to the polynucleotide-binding protein, or the side chain region does not bind to the polynucleotide-binding protein.

## Description

### CROSS-REFERENCE TO RELATED APPLICATIONS

The present application claims priority to Chinese Patent Application No. 202111398053.X entitled "ADAPTER, COMPLEX, SINGLE-STRANDED MOLECULE, KIT, METHOD, AND USE THEREOF" filed on November 23, 2021, which is incorporated herein by reference in its entirety.

### TECHNICAL FIELD

The present application relates to nanopore sequencing technology, particularly to an adapter for characterizing biomolecules, a nucleic acid-protein complex, a single-stranded molecule, a method for characterizing biomolecules, a kit, and use thereof.

### BACKGROUND

In nanopore sequencing, it is necessary to connect a biomolecule to be tested to a sequencing linker (or adapter), and the biomolecule to be tested is guided to move through a nanopore by means of the sequencing linker or a sequencing structure connected to the sequencing linker. Generally, the sequencing linker is provided with a segment of single-stranded DNA sequence that binds to a tether distributed in the vicinity of the nanopore, so that the biomolecule to be tested can be targeted to the vicinity of the nanopore. This segment of single-stranded DNA sequence is referred to as a side chain.

In the preparation process of the sequencing linker, a polynucleotide-binding protein for assisting the biomolecule to be tested to complete the characterization needs to bind to a designated region of the sequencing linker, and the polynucleotide-binding protein may also bind to the side chain, potentially affecting the connection between the sequencing linker and the tether. In the related art, the polynucleotide-binding protein moiety bound to the side chain can be removed using a solution of potassium chloride and adenosine triphosphate, and then the sequencing linker can be used for sequencing. However, treatment with the solution of potassium chloride and adenosine triphosphate increases the preparation time and reduces the detection efficiency.

### SUMMARY

One of the objectives of the embodiments of the present application is to provide an adapter for characterizing biomolecules, a nucleic acid-protein complex, a single-stranded molecule, a method for characterizing biomolecules, a kit, and use thereof, wherein the side chain region has a weaker affinity for the polynucleotide-binding protein, such that the side chain region is prevented from binding to the polynucleotide-binding protein, thereby increasing the binding rate of the polynucleotide-binding protein to a main body strand and thus improving the efficiency of characterizing the biomolecule.

The present application provides the following technical solutions:
In a first aspect, provided is an adapter comprising a main body strand and a connecting strand, wherein the main body strand is used for binding to a biomolecule to be tested and a polynucleotide-binding protein; the connecting strand comprises a connecting region and a side chain region, the connecting region being connected to the main body strand, and the side chain region being used for connecting to an external connector; the binding ability of the side chain region to the polynucleotide-binding protein is weaker than the binding ability of the main body strand to the polynucleotide-binding protein, or the side chain region does not bind to the polynucleotide-binding protein.

In a second aspect, an embodiment of the present application provides a nucleic acid-protein complex comprising the adapter according to the first aspect and a polynucleotide-binding protein, wherein the polynucleotide-binding protein is bound to the main body strand.

In a third aspect, an embodiment of the present application provides a single-stranded molecule comprising the connecting strand according to the first aspect.

In a fourth aspect, an embodiment of the present application provides a method for characterizing biomolecules, comprising:
(a) binding a polynucleotide-binding protein and a biomolecule to be tested to the adapter according to the first aspect to form a first complex;
(b) contacting the first complex with a transmembrane nanopore provided with an external connector or a molecular membrane where the transmembrane nanopore is located; and
(c) allowing the biomolecule to be tested to pass through the transmembrane nanopore and move relative to the transmembrane nanopore, and obtaining one or more measurements representative of one or more characteristics of the biomolecule to be tested, thereby characterizing the biomolecule to be tested.

In a fifth aspect, an embodiment of the present application provides a kit comprising the adapter according to the first aspect, the nucleic acid-protein complex according to the second aspect, or the single-stranded molecule according to the third aspect.

In a sixth aspect, an embodiment of the present application provides use of the adapter according to the first aspect, the nucleic acid-protein complex according to the second aspect, the single-stranded molecule according to the third aspect, the method of controlling the nucleic acid-protein complex to pass through the transmembrane nanopore according to the fourth aspect, or the kit according to the fifth aspect in characterizing a biomolecule.

The technical solutions provided by the embodiments of the present application at least bring the following beneficial effects:

In the adapter provided by the embodiment of the present application, because the side chain region has a weaker affinity for the polynucleotide-binding protein, the side chain region of the adapter is prevented from binding to the polynucleotide-binding protein; the side chain region may also be connected to the external connector without altering the sequence or structure of the existing external connector, allowing the adapter to be enriched in the vicinity of the molecular membrane with the transmembrane nanopore.

It should be understood that both the above general description and the following detailed description are merely illustrative and explanatory, and are not intended to limit the present application.

### BRIEF DESCRIPTION OF THE DRAWINGS

The accompanying drawings, which are incorporated in and constitute a part of this specification, illustrate embodiments consistent with the present application and, together with the specification, serve to explain the principles of the present application and are not intended to improperly limit the present application.
FIG. 1 shows a schematic diagram of the structure of an adapter according to an embodiment of the present application;
FIG. 2 shows a schematic diagram of the structure of an adapter according to another embodiment of the present application;
FIG. 3 shows a gel electropherogram of Comparative Example 1 of the present application;
FIG. 4 shows a gel electropherogram of Example 1 of the present application;
FIG. 5 shows a sequencing signal diagram for nanopore sequencing by an adapter according to an embodiment of the present application; and
FIG. 6 is a gel electropherogram after binding the side chain region to the polynucleotide-binding protein according to an embodiment of the present application.

In order to enable those skilled in the art to better understand the technical solutions of the present application, the technical solutions in the embodiments of the present application will be clearly and completely described below with reference to the accompanying drawings. It should be understood that the specific embodiments described herein are intended only to illustrate the present application, and not to limit the present application. For those skilled in the art, the present application can be implemented without some of these specific details. The following description of the embodiments is intended only to provide a better understanding of the present application by illustrating examples of the present application.

It should be noted that the terms "first", "second", and so on in the specification and claims of the present application and in the drawings described above are used for distinguishing between similar objects and not necessarily for describing a specific order or sequence. It should be understood that the data used in this manner can be interchangeable under appropriate circumstances, such that the embodiments of the present application described herein are capable of being implemented in an order other than those illustrated or described herein. The modes of implementation described in the following exemplary embodiments do not represent all modes of implementation consistent with the present application. Rather, they are merely examples consistent with certain aspects of the present application as detailed in the appended claims.

Based on the background, the applicant has found that in the preparation process of the sequencing linker, the polynucleotide-binding protein bound to the side chain needs to be treated with a solution of potassium chloride and adenosine triphosphate to remove the polynucleotide-binding protein bound to the side chain, thereby increasing the preparation time of the sequencing adapter and reducing the detection efficiency.

In order to solve the above problems, an embodiment of the present application provides an adapter, with details available in the following embodiments.

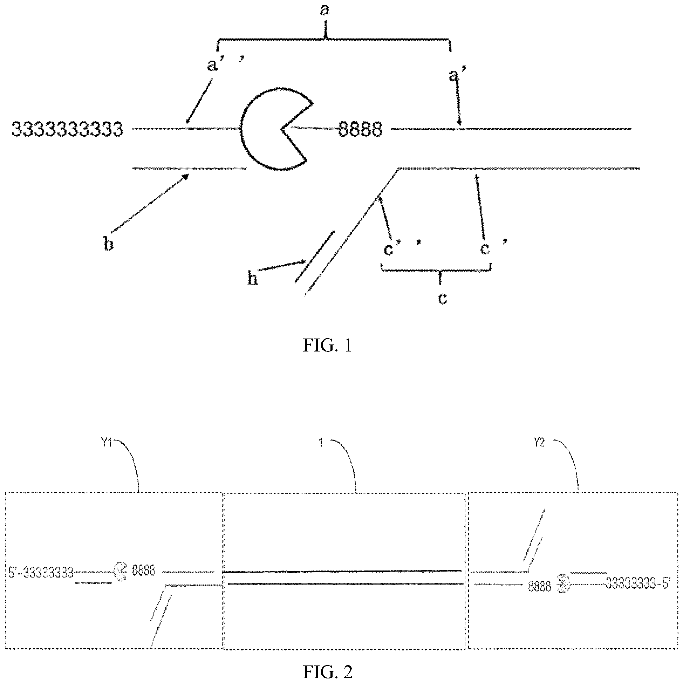

Referring to FIG. 1, an embodiment of the present application provides an adapter comprising a main body strand and a connecting strand, wherein the main body strand is used for binding to a biomolecule to be tested and a polynucleotide-binding protein; the connecting strand comprises a connecting region and a side chain region, the connecting region being connected to the main body strand, and the side chain region being used for connecting to an external connector; the binding ability of the side chain region to the polynucleotide-binding protein is weaker than the binding ability of the main body strand to the polynucleotide-binding protein, or the side chain region does not bind to the polynucleotide-binding protein.

The adapter provided by the present application is useful for biomolecule characterization, and in particular for nanopore sequencing-based biomolecule characterization. The biomolecule may specifically be one of a polynucleotide, a polypeptide, a polysaccharide, and a lipid. The biomolecules to be tested can sequentially pass through the transmembrane nanopore on the molecular membrane under the action of an electrical potential, and the passage of the biomolecules to be tested through the transmembrane nanopore can cause changes in the electrical signals, based on which the characterization information of the biomolecules to be tested can be further obtained. For example, size information, sequence information, identity information, modification information, and the like of the biomolecule to be tested are obtained based on the current change information. The transmembrane nanopore may be a through hole disposed in the direction of the thickness of the molecular membrane to communicate both sides of the molecular membrane. The transmembrane nanopore may specifically be a protein pore, a polynucleotide pore, a solid-state pore, or the like, and the present application does not limit the specific form in which the transmembrane nanopore is formed.

The polynucleotide-binding protein may be a protein that is capable of binding to a polynucleotide and assisting in its movement relative to a transmembrane nanopore, such as passage through the pore. The protein typically interacts with the polynucleotide and modifies at least one property of the polynucleotide. Before the biomolecule to be tested passes through the transmembrane nanopore, it is necessary to first bind the adapter to the polynucleotide-binding protein to form a nucleic acid-protein complex, and then bind the nucleic acid-protein complex to the biomolecule to be tested, so that the biomolecule to be tested can pass through the transmembrane nanopore by the assistance of the polynucleotide-binding protein. The polynucleotide-binding protein may modify the nucleic acid-protein complex by cleaving a polynucleotide to form single nucleotides or shorter strands of nucleotides, such as dinucleotides or trinucleotides. The polynucleotide-binding protein may modify a polynucleotide by directing the polynucleotide or moving the polynucleotide to a specific position, for example, by controlling the movement of the polynucleotide. The number of polynucleotide-binding proteins connected to the main body strand may be plural, and the types of the plural polynucleotide-binding proteins may be different. The polynucleotide-binding protein may be any one or more of a polymerase, a helicase, an endonuclease, an exonuclease, a DNA protease, and a topoisomerase. Those skilled in the art may set the number, type and connection position of the polynucleotide-binding proteins to be connected to the main body strand as desired.

The polynucleotide-binding protein is preferably a helicase, and any helicase may be used in the present application. The helicase may be (a) Hel308 helicase, RecD helicase, XPD helicase, or Dda helicase; (b) a helicase derived from any of the helicases described in (a); or (c) any combination of the helicases described in (a) and/or (b).

In the adapter, the main body strand is used for binding to the biomolecule to be tested, and the connecting strand is used for binding to a connector provided on the molecular membrane so as to bring the biomolecule to be tested close to the molecular membrane. In the process of characterizing biomolecules, the more biomolecules to be tested that are free in the vicinity of the transmembrane nanopore, the higher the efficiency of the biomolecules to be tested passing through the transmembrane nanopore, thus demonstrating higher detection efficiency. Therefore, by providing the connector in the transmembrane nanopore or in the vicinity of the transmembrane nanopore, and connecting the connector to the connecting strand, the efficiency of the biomolecules to be tested passing through the transmembrane nanopore can be improved. If the molecular membrane is an amphiphilic layer, such as a copolymer membrane or a lipid bilayer, the connector comprises a polypeptide anchor and/or a hydrophobic anchor embedded in the molecular membrane or on the surface of the molecular membrane, as well as a tether connected to the polypeptide anchor and/or the hydrophobic anchor. The hydrophobic anchor is preferably a lipid, a fatty acid, a sterol, a carbon nanotube, a polypeptide, a protein, or an amino acid, such as cholesterol, palmitate, or tocopherol. The tether is a polynucleotide strand with a certain specificity, and the side chain region and the tether in the connector may be connected through complementary base pairing, thereby achieving the connection between the connecting strand and the connector.

Nucleic acid analogues refer to nucleic acid-derived substances that have properties similar to those of RNA and DNA. RNA and DNA are composed primarily of phosphates, pentose sugars, and bases, while nucleic acid analogues have at least one of these components substituted with some other substance. The nucleic acid analogues include, for example, peptide nucleic acid (PNA), morpholino nucleic acid (MNA), bridged nucleic acid (BNA), locked nucleic acid (LNA), glycol nucleic acid (GNA), threose nucleic acid (TNA), etc. The side chain region may be specifically configured as ribonucleotides (RNA) and/or a nucleic acid analogue to retain the original base specificity, allowing it to still bind to the connector while reducing its binding ability to the polynucleotide-binding protein. Of course, the side chain region may also be modified deoxyribonucleotides (DNA).

In one embodiment, the side chain region of the connecting strand comprises a nucleic acid analogue; the side chain region of the connecting strand is non-covalently connected to the external connector, and the connecting region of the connecting strand is non-covalently connected to the main body strand. Optionally, the side chain region is connected to the external connector through complementary base pairing to form a stable double-stranded structure, so that the connection between the connecting strand and the connector is stable. The connecting region is connected to the main body strand through complementary base pairing to form a stable double-stranded structure, so that the connection between the connecting strand and the main body strand is stable. Optionally, the nucleic acid analogue includes any one of or any combination of two or more of a peptide nucleic acid (PNA), a glycerol nucleic acid (GNA), a threose nucleic acid (TNA), a locked nucleic acid (LNA), and a bridged nucleic acid (BNA), that is, the side chain region is formed by connecting a plurality of peptide nucleic acids (PNAs), glycerol nucleic acids (GNAs), threose nucleic acids (TNAs), bridged nucleic acids, or locked nucleic acids (LNAs), such that the side chain region has a certain specificity. The base sequence of the nucleic acid analogue may be designed with reference to the sequence of the connector provided on the molecular membrane. In another embodiment, the side chain region of the connecting strand comprises a ribonucleotide (RNA), and the ribonucleotide in the side chain region of the connecting strand includes a 2'-modified ribonucleotide in which the hydroxyl group at the 2'-position of the ribose is modified to reduce the binding ability to the polynucleotide-binding protein. Optionally, the side chain region comprises a 2'-alkoxy-modified ribonucleotide and/or a 2'-methoxy-modified ribonucleotide so as to facilitate artificial synthesis.

The main body strand is used for connecting to the biomolecule to be tested, and the main body strand is provided with a polynucleotide-binding protein binding site, a spacer site, etc., so as to further control the passage of the biomolecule to be tested through the transmembrane nanopore, and the speed of the passage through the transmembrane nanopore, etc. The main body strand may specifically be single-stranded or double-stranded; optionally, at least a portion of the main body strand is double-stranded. The main body strand is entirely or primarily composed of deoxyribonucleotides (DNA), and a ribonucleotide or a nucleic acid analogue may be provided in the main body strand as required or a modification may be added thereto as required. Since the main body strand is also used for binding to the polynucleotide-binding protein, in order to ensure that the binding ability of the polynucleotide-binding protein to the main body strand is greater than the binding ability of the polynucleotide-binding protein to the side chain region, optionally, the portion of the main body strand that is used for binding to the polynucleotide-binding protein is entirely configured as deoxyribonucleotides.

In one embodiment, the adapter further comprises an antisense strand complementarily base-paired with the main body strand, and the main body strand comprises a first portion complementarily base-paired with the connecting region of the connecting strand, a loading portion used for binding to the polynucleotide-binding protein, a second portion complementarily base-paired with the antisense strand, and a binding portion used for binding to the biomolecule to be tested, wherein the loading portion comprises a polynucleotide. For example: in FIG. 1, strands *a*, *b* and *c* constitute the adapter, where the illustrated strand *a* is the main body strand, the illustrated strand *b* is the antisense strand, the illustrated strand c is the connecting strand, the illustrated region *c'* is the connecting region, the illustrated region *c"* is the side chain region, the illustrated region *a'* is the first portion of the main body strand, and the illustrated region *a"* is the second portion of the main body strand. Strands *a* and *b* are both deoxyribonucleotide (DNA) single strands, the region *c'* is deoxyribonucleotides (DNA), and the region *c"* is ribonucleotides (RNA) or a nucleic acid analogue, wherein the region *a'* is complementary to the region *c'*, the region *a"* is complementary to the strand b, and one end of the region c' distal to the loading portion is provided with a binding portion which can be specifically connected to the biomolecule to be tested through TA ligation.

The main body strand and the antisense strand are linear molecules composed primarily of polynucleotides. It will be appreciated by those skilled in the art that, in order to meet the specific needs of characterizing the biomolecule, the main body strand and the antisense strand may also be modified, and connected to a desired chemical group, etc. For example, in the main body strand *a* shown in FIG. 1, "3333333333" is a C3 spacer modification, and "8888" is a C18 spacer modification. Of course, it is also possible to include a tag sequence in any strand of the adapter to distinguish between different adapters. Therefore, in the context of the present application, the "DNA" single strand should be understood in its broadest sense. Similarly, the connecting strand may also be modified on the ribonucleotide or nucleic acid analogue, and connected to a desired chemical group, etc.

A technical solution of an embodiment of the present application provides an adapter, wherein because the side chain region is ribonucleotides (RNA) or a nucleic acid analogue, it has a weaker affinity for the polynucleotide-binding protein, preventing the side chain region of the adapter from binding to the polynucleotide-binding protein; meanwhile, because the side chain region is ribonucleotides (RNA) or a nucleic acid analogue, the side chain region still has bases, and the side chain region may also be connected to the external connector through complementarity base pairing without altering the sequence or structure of the existing external connector, allowing the adapter to be enriched in the vicinity of the molecular membrane with the transmembrane nanopore.

During the process where the polynucleotide-binding protein assists the biomolecule to be tested to pass through the transmembrane nanopore, the polynucleotide-binding protein may move in a 3' to 5' direction or in a 5' to 3' direction. When the polynucleotide-binding protein is an enzyme that moves in the 3' to 5' direction, the 3' end of the loading portion is connected to the 5' end of the connecting region; when the polynucleotide-binding protein is an enzyme that moves in the 5' to 3' direction, the 5' end of the loading portion is connected to the 3' end of the connecting region.

It should be noted that before the adapter provided by the present application is used for the characterization of the biomolecule to be tested, the main body strand, the antisense strand, and the connecting strand need to be first prepared, then mixed according to a certain proportion and annealed to form the adapter. The adapter binds to the polynucleotide-binding protein to form a nucleic acid-protein complex, which also needs to bind to the biomolecule to be tested, so as to control the speed at which the biomolecule to be tested passes through the transmembrane nanopore by means of the polynucleotide-binding protein. Specifically, the first portion of the main body strand and the connecting region of the connecting strand may bind to the two complementary double strands of the biomolecule to be tested, respectively; for example, the region *a'* and the region *c'* shown in FIG. 1 bind to the biomolecule to be tested, then the side chain region *c"* is connected to the connector through complementary base pairing, and under the control of the polynucleotide-binding protein and the action of an electrical potential, the biomolecule to be tested passes through the transmembrane nanopore on the molecular membrane. It will be appreciated by those skilled in the art that in the present application, the specific base sequences in the main body strand, the antisense strand, and the connecting strand may be configured according to different sequencing platforms, different sequencing requirements, and the requirements of the molecule to be tested.

Further, the adapter may be prepared by the following method: designing and synthesizing the main body strand, the antisense strand and the connecting strand described above, and annealing the same to form the adapter. In this method, the modification process of each strand may be completed before annealing, and the obtained side chain region is modified using a chemical synthesis method. Specifically, the modification may be performed using a solid phase method. The specific solid phase method belongs to the related art and will not be described in detail here.

In one embodiment, when the connector is cholesterol with a tether, the side chain region may be complementarily base-paired with the tether. After the side chain region is complementarily base-paired with the tether, it may be accessed to the molecular membrane of the transmembrane nanopore based on the cholesterol with the tether. The side chain region of the connecting strand is a region of high charge density, and the side chain region of the connecting strand may wander in the vicinity of the transmembrane nanopore. When the side chain region is complementarily base-paired with the tether, the biomolecule to be tested connected to the adapter enters the transmembrane nanopore under the action of an external electrical potential, so as to realize the characterization of the biomolecule to be tested through a change in the electrical signal.

In one example, nanopore sequencing is performed based on a complex of the adapter, and in order to understand the specific structure of the complex of the adapter, an embodiment of the present application further provides a nucleic acid-protein complex, with details available in the following embodiment.

The nucleic acid-protein complex according to an embodiment of the present application is composed of the adapter and the polynucleotide-binding protein described above, wherein the polynucleotide-binding protein binds to the loading portion of the main body strand, that is, the polynucleotide-binding protein may bind to the strand *a* in FIG. 1.

Therefore, in this complex, the side chain region has a weak affinity for the polynucleotide-binding protein, and the loading portion has a strong affinity for the polynucleotide-binding protein, so that the polynucleotide-binding protein binds only to the loading portion, thereby eliminating the need to elute the polynucleotide-binding protein bound to the side chain with a solution of potassium chloride and adenosine triphosphate, and improving the preparation efficiency; the side chain region is mostly or entirely retained for binding to the connector, enhancing the anchoring effect of the adapter.

In one example, the polynucleotide-binding protein is selected from any one of a polymerase, a helicase, an endonuclease, an exonuclease, and a DNA-binding protease.

In one embodiment, the polynucleotide-binding protein is set forth in SEQ ID NO. 1. The polynucleotide-binding protein may specifically bind before the C18 spacer modification as shown in FIG. 1.

The prepared nucleic acid-protein complex needs to be connected to the biomolecule to be tested so as to guide the biomolecule to be tested to pass through the transmembrane nanopore. Two nucleic acid-protein complexes may be symmetrically connected to two ends of a double-stranded structure of a biomolecule to be tested. Referring to FIG. 2, in one embodiment, the binding portion of the main body strand of the nucleic acid-protein complex Y1 is connected to the 5' end of one strand in the biomolecule 1 to be tested, and the connecting region of the connecting strand of the nucleic acid-protein complex Y2 is connected to the 3' end of the same strand in the biomolecule 1 to be tested; similarly, the connecting region of the connecting strand of the nucleic acid-protein complex Y1 is connected to the 3' end of the other strand in the biomolecule 1 to be tested, and the binding portion of the main body strand of the nucleic acid-protein complex Y2 is connected to the 5' end of the same strand in the biomolecule 1 to be tested.

A technical solution of an embodiment of the present application provides a nucleic acid-protein complex, wherein the nucleic acid-protein complex is composed of the adapter and the polynucleotide-binding protein described above. Therefore, in the complex, the side chain region has a weak affinity for the polynucleotide-binding protein, so that the polynucleotide-binding protein binds only to the loading portion, thereby eliminating the need to elute the polynucleotide-binding protein bound to the side chain with a solution of potassium chloride and adenosine triphosphate, and improving the preparation efficiency; the side chain region is mostly or entirely retained for binding to the connector, enhancing the anchoring effect of the adapter.

The above embodiments describe adapters, as well as nucleic acid-protein complexes that are used to characterize biomolecules. In order to describe how to characterize biomolecules, an embodiment of the present application provides a method for characterizing biomolecules, with details available in the following embodiment.

An embodiment of the present application provides a method of controlling a nucleic acid-protein complex to pass through a transmembrane nanopore, which may specifically comprise the following steps:
S10, binding a polynucleotide-binding protein and a biomolecule to be tested to the aforementioned adapter to form a first complex, that is,
contacting a pre-prepared adapter with a polynucleotide-binding protein to allow the polynucleotide-binding protein to bind to the main body strand of the adapter, and then connecting the main body strand to a biomolecule to be tested to form a first complex, wherein in some embodiments of the present application, the biomolecule may specifically be one of a polynucleotide, a polypeptide, a polysaccharide, and a lipid;
S20, contacting the first complex with a transmembrane nanopore provided with an external connector or a molecular membrane where the transmembrane nanopore is located, wherein
in some embodiments of the present application, the connector may be pre-inserted on the molecular membrane with the nanopore; the connector has a naked tether, which may be bound to a side chain region of the adapter; in one example, the connector may be cholesterol with a tether, such as strand *h* in FIG. 1;
S30, allowing the biomolecule to be tested to pass through the transmembrane nanopore and move relative to the transmembrane nanopore, and obtaining one or more measurements representative of one or more characteristics of the biomolecule to be tested, thereby characterizing the biomolecule to be tested.

In step S30, since the tether is embedded in the molecular membrane with the transmembrane nanopore, the first complex formed in S30 may drive the enrichment of the biomolecule to be tested on the surface of the molecular membrane. A voltage is applied on both sides of the molecular membrane, such that the biomolecule to be tested passes through the transmembrane nanopore under the action of an electrical potential; meanwhile, the pore crossing speed is controlled by the polynucleotide-binding protein, so as to realize the characterization of the biomolecule to be tested through a change in the electrical signal. In one example, the polynucleotide-binding protein is selected from any one or more of a helicase, a polymerase, an endonuclease, an exonuclease, and a DNA-binding protease.

The present application further provides a kit comprising the adapter as described above; or the nucleic acid-protein complex as described above; or the single-stranded molecule as described above. The single-stranded molecule is the aforementioned connecting strand. The kit provided by the embodiment of the present application, due to the adoption of the adapter provided by any one of the above embodiments, achieves the same technical effects, which will not be described herein again.

Specifically, the nanopore sequencing kit further comprises a buffer for storing the adapter or the nucleic acid-protein complex, wherein the buffer may include an L fragment buffer (LFB), an S fragment buffer (SFB), a ligation buffer (LNB), an elution buffer (EB), and a sequencing buffer (SQB); it will be appreciated by those skilled in the art that the nanopore sequencing kit provided by the present application may further comprise one or more of independently packaged items such as a sequencing tether (SQT), a DNA control strand, and a loading bead (LB), but is not limited thereto, so as to facilitate a user to prepare a sample that can be directly loaded onto the machine.

The present application further provides use of the adapter, the nucleic acid-protein complex, or the method of controlling the nucleic acid-protein complex to pass through the transmembrane nanopore, or the kit described above in characterizing a biomolecule. Due to the adoption of the adapter provided by any one of the above embodiments, the same technical effects are achieved, which will not be described herein again.

To verify the effects achievable by the above embodiments, examples and comparative examples are provided below for illustration:

### Example 1: Preparation of Nucleic Acid-Protein Complexes

A main body strand, an antisense strand, and a connecting strand were separately synthesized and then subjected to an annealing treatment at a ratio of 1: 1.1:1.1 to form an adapter. The annealing treatment specifically involved slowly cooling from 95 °C to 25 °C, with a cooling rate of not exceeding 0.1 °C/s. The final annealing system included 160 mM HEPES 7.0 and 200 mM NaCl, with the final concentration of the main body strand being 4-8 µM.

500 nM adapter, T4 helicase at 6 times the molar amount, and 1.5 mM TMAD (tetramethylazodicarboxamide) were mixed and then incubated at room temperature for 30 min to prepare the nucleic acid-protein complex, wherein the helicase could be T4 Dda M1G/E94C/C109A/C136A/A360C (set forth in SEQ ID NO. 1).

The nucleic acid-protein complex was loaded onto a DNAPac PA200 column and purified with an elution buffer to wash off the enzyme that had not bound to the adapter from the column.

Then, the nucleic acid-protein complex was eluted with a mixture of buffer A and buffer B in a volume 10 times that of the column, and then the main elution peak was pooled, measured for the concentration, and run on a TBE PAGE gel at 160V for 40 min, wherein buffer A: 20 mM Na-CHES, 250 mM NaCl, 4% (W/V) glycerol, pH 8.6; and buffer B: 20 mM Na-CHES, 1 M NaCl, 4% (W/V) glycerol, pH 8.6.

The sequence of the adapter is as follows:
The main body strand is set forth in SEQ ID NO. 2; main body strand S1: 5'-333333333333333333333333333333-**GCGGAGTCAAACGGTAGAAGTCG**TTTTTTTTTT-8888-ACTGCTCATTCGGTCCTGCTGAC T-3';
The antisense strand is set forth in SEQ ID NO. 3; antisense strand S2: 5'-**CGACTTCTACCGTTTGACTCCGC**-3';
The connecting strand is set forth in SEQ ID NO. 4; connecting strand YB: 5'-GTCAGCAGGACCGAATGA /i2OMeG//i2OMeC//i2OMeA//i2OMeG//i2OMeU//i2OMeA//i2OMeG//i2OMeU//i2OMeC//i2O MeC//i2OMeA//i2OMeG//i2OMeC//i2OMeA//i2OMeC//i2OMeC//i2OMeG//i2OMeA//i2OMeC// i2OMeC/-3', wherein i2Ome is 2"-O-methyl RNA, i.e., 2'-methoxy-modified RNA.

Comparative Example 1: The procedures were performed in substantially the same manner as those in Example 1 using the same adapter main body strand S 1 and antisense strand S2 as in Example 1, except that the connecting strand YC was a DNA strand.

Connecting strand YC: 5'-GTCAGCAGGACCGAATGAGCAGTAGTCCAGCACCGACC-3'.

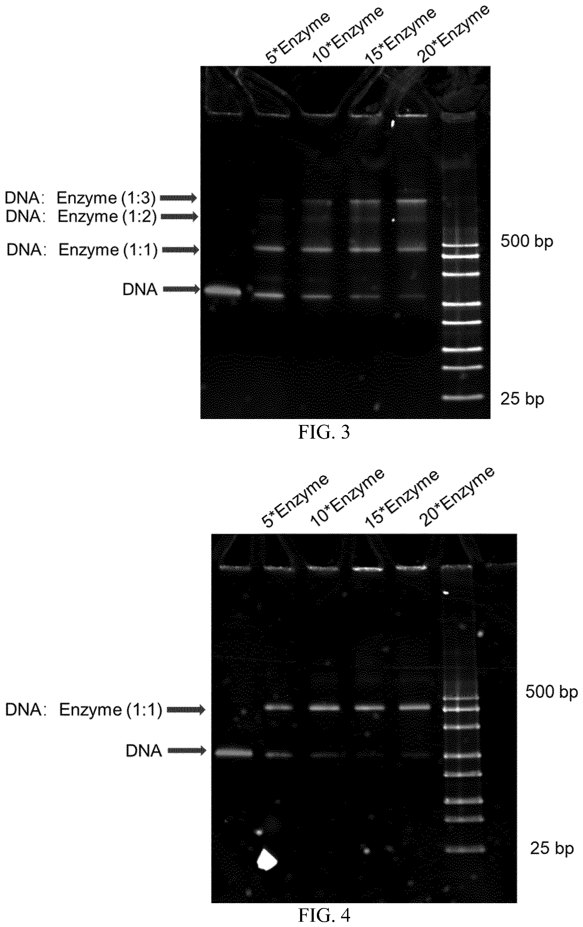

Referring to FIGs. 3 and 4, FIG. 3 is a TBE PAGE gel diagram of Comparative Example 1, and from left to right in FIG. 3, lane 1 is for the adapter of Comparative Example 1; lane 2 is for the nucleic acid-protein complex prepared with the adapter and 5× polynucleotide-binding protein; lane 3 is for the nucleic acid-protein complex prepared with the adapter and 10× polynucleotide-binding protein; lane 4 is for the nucleic acid-protein complex prepared with the adapter and 15× polynucleotide-binding protein; lane 5 is for the nucleic acid-protein complex prepared with the adapter and 20× polynucleotide-binding protein; and lane 6 is for Maker. FIG. 4 is a TBE PAGE gel diagram of Example 1, and from left to right in FIG. 4, lane 1 is for the adapter of Example 1; lane 2 is for the nucleic acid-protein complex prepared with the adapter and 5× polynucleotide-binding protein; lane 3 is for the nucleic acid-protein complex prepared with the adapter and 10× polynucleotide-binding protein; lane 4 is for the nucleic acid-protein complex prepared with the adapter and 15× polynucleotide-binding protein; lane 5 is for the nucleic acid-protein complex prepared with the adapter and 20× polynucleotide-binding protein; and lane 6 is for Maker.

It can be seen from FIG. 3 that lanes 2 to 5 each corresponded to multiple bands, with the size of each band being larger than that of the adapter of Comparative Example 1 in lane 1; based on the size of the corresponding multiple bands, it can be inferred that the multiple bands corresponded, in order, to the complexes obtained by binding the adapter in Comparative Example 1 to 1, 2, and 3 polynucleotide-binding proteins, respectively. In addition, with the increase in the amount of the added polynucleotide-binding protein, the amount of complexes bound with multiple polynucleotide-binding proteins significantly increased. Compared with FIG. 3, it can be seen from FIG. 4 that lanes 2 to 5 corresponded only to one band larger than that of the adapter of Example 1 in lane 1, and this band had a size similar to that corresponding to the complex obtained by binding the adapter to one polynucleotide-binding protein as shown in FIG. 3. In addition, with the increase in the amount of the added polynucleotide-binding protein, the band with that size became significantly brighter, indicating that with the increase in the amount of polynucleotide-binding protein, the nucleic acid-protein complexes significantly increased. Therefore, the adapter provided by the present application can efficiently bind to polynucleotide-binding proteins, avoiding the situation where multiple polynucleotide-binding proteins bind to one adapter.

### Example 2: On-Machine Test of Nucleic Acid-Protein Complexes

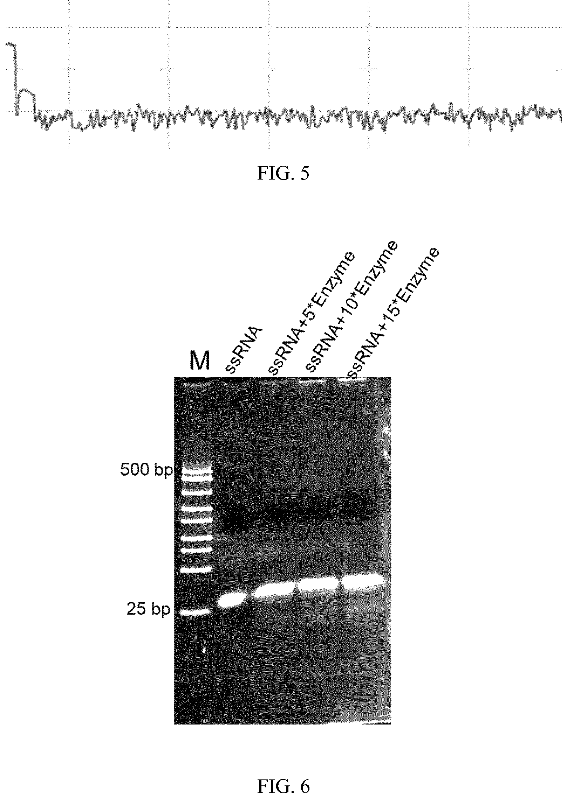

A library with a length of 10 kb was prepared by an end-repairing method, which was then ligated with the nucleic acid-protein complex prepared in Example 1 for library construction. Finally, sequencing was performed on QNome-9604 from Qitan Technology Ltd., with the actual sequencing signals shown in FIG. 5.

It can be seen from FIG. 5 that using the nucleic acid-protein complex prepared in Example 1, excellent sequencing results were obtained, thereby demonstrating that the nucleic acid-protein complex prepared in Example 1 could normally bind to the connector and effectively guide the biomolecule to be tested for through-pore sequencing.

### Example 3: Verification of Binding of Side Chain Region to Polynucleotide-Binding Protein

ssRNA was synthesized with a sequence of 5'-/rU//rU//rU//rU//rU//rU//rU//rU//rU//rU//rU//rU/-3'.

The synthesized ssRNA was mixed with a polynucleotide-binding protein (specifically T4 Dda M1G/E94C/C109A/C136A/A360C) at ratios of 1:5, 1:10, and 1:15, respectively, and the mixtures were incubated at room temperature for 30 min and then run on a TBE PAGE gel at 160 V for 25 min.

Referring to FIG. 6, lane 1 is for Maker; lane 2 is for ssRNA; lane 3 is for ssRNA incubated with 5× polynucleotide-binding protein; lane 4 is for ssRNA incubated with 10× polynucleotide-binding protein; and lane 5 is for ssRNA incubated with 15× polynucleotide-binding protein. It can be seen from FIG. 6 that lanes 3 to 5 each corresponded to multiple bands, indicating that the polynucleotide-binding protein did not bind to the ssRNA, demonstrating that the RNA sequence had a low degree of binding to the polynucleotide-binding protein.

It should also be noted that the exemplary examples mentioned herein describe some methods, complexes or kits based on a series of steps. However, the present application is not limited to the order of the above steps, that is, the steps may be performed in the order mentioned in the examples or in an order different from the order in the examples, or several steps may be performed simultaneously.

What is mentioned above is merely specific embodiments of the present application, and it is clear to those skilled in the art that, for the sake of convenience and brevity in description, the methods, the complexes and the kits described above may refer to the corresponding descriptions in the aforementioned adapter embodiments, which will not be described herein again. It should be understood that the protection scope of the present application is not limited to these embodiments, and any person skilled in the art can easily think of various equivalent modifications or substitutions within the technology scope disclosed in the present application, and these modifications or substitutions shall fall within the protection scope of the present application.

## Claims

1. An adapter for characterizing biomolecules, comprising:
a main body strand, used for binding to a biomolecule to be tested and a polynucleotide-binding protein; and
a connecting strand, comprising a connecting region and a side chain region, wherein the connecting region is connected to the main body strand, the side chain region is used for connecting to an external connector, and the binding ability of the side chain region to the polynucleotide-binding protein is weaker than the binding ability of the main body strand to the polynucleotide-binding protein, or the side chain region does not bind to the polynucleotide-binding protein.

2. The adapter according to claim 1, wherein the side chain region of the connecting strand comprises a ribonucleotide (RNA) and/or a nucleic acid analogue; and/or
the side chain region of the connecting strand is connected to the external connector non-covalently, preferably through complementary base pairing; and/or
the connecting region of the connecting strand is connected to the main body strand non-covalently, preferably through complementary base pairing.

3. The adapter according to claim 2, wherein the ribonucleotide in the side chain region of the connecting strand comprises a 2'-modified ribonucleotide; preferably, the side chain region comprises a 2'-alkoxy-modified ribonucleotide and/or a 2'-methoxy-modified ribonucleotide;
the nucleic acid analogue comprises any one of or any combination of two or more of a peptide nucleic acid (PNA), a glycerol nucleic acid (GNA), a threose nucleic acid (TNA), a locked nucleic acid (LNA), and a bridged nucleic acid (BNA).

4. The adapter according to any one of claims 1 to 3, wherein the main body strand comprises a first portion connected to the connecting region of the connecting strand, a loading portion used for binding to the polynucleotide-binding protein, and/or a binding portion used for binding to the biomolecule to be tested;
the loading portion comprises a polynucleotide.

5. The adapter according to any one of claims 1 to 4, wherein the polynucleotide-binding protein is an enzyme that moves in a 3' to 5' direction, and the 3' end of the loading portion is connected to the 5' end of the connecting region; or
the polynucleotide-binding protein is an enzyme that moves in a 5' to 3' direction, and the 5' end of the loading portion is connected to the 3' end of the connecting region; and/or
the polynucleotide-binding protein is selected from any one of or any combination of two or more of a polymerase, a helicase, an endonuclease, an exonuclease, and a DNA-binding protease.

6. A nucleic acid-protein complex, comprising the adapter according to any one of claims 1 to 5 and a polynucleotide-binding protein, wherein the polynucleotide-binding protein is bound to the main body strand.

7. A single-stranded molecule, comprising the connecting strand according to any one of claims 1 to 5.

8. A method for characterizing biomolecules, comprising:
(a) binding a polynucleotide-binding protein and a biomolecule to be tested to the adapter according to any one of claims 1 to 5 to form a first complex;
(b) contacting the first complex with a transmembrane nanopore provided with an external connector or a molecular membrane where the transmembrane nanopore is located; and
(c) allowing the biomolecule to be tested to pass through the transmembrane nanopore and move relative to the transmembrane nanopore, and obtaining one or more measurements representative of one or more characteristics of the biomolecule to be tested, thereby characterizing the biomolecule to be tested.

9. A kit for characterizing biomolecules, comprising the adapter according to any one of claims 1 to 5, the nucleic acid-protein complex according to claim 6, or the single-stranded molecule according to claim 7, preferably further comprising a polynucleotide-binding protein and/or a membrane component of the molecular membrane according to claim 8 and/or the external connector according to claim 8.

10. Use of the adapter according to claims 1 to 5, the nucleic acid-protein complex according to claim 6, the single-stranded molecule according to claim 7, the method for characterizing biomolecules according to claim 8, or the kit according to claim 9 in characterizing a biomolecule or in preparing a product for characterizing a biomolecule, wherein
preferably, the biomolecule comprises a polynucleotide, a polypeptide, a polysaccharide, or a lipid; more preferably, the polynucleotide comprises a DNA or an RNA; and/or the characterized characteristic is selected from: (i) a length of the polynucleotide; (ii) an identity of the polynucleotide; (iii) a sequence of the polynucleotide; (iv) a secondary structure of the polynucleotide; and (v) whether the polynucleotide is modified.
